# EUROPÄISCHE PATENTSCHRIFT

(11) **EP 1 135 193 B1**
(45) Veröffentlichungstag und Bekanntmachung des Hinweises auf die Patenterteilung: **30.10.2002**
(21) Anmeldenummer: 99964421.4
(22) Anmeldetag: 06.12.1999
(51) Int. Cl.: A61P 35/00, A61K 31/135, A61K 31/66, A61K 31/575

(54) **MITTEL ZUR TUMORTHERAPIE AUF LIPOSOMENBASIS, DIE TAMOXIFEN ENTHALTEN**
AGENTS PROVIDED FOR TREATING TUMORS, BASED ON LIPOSOMES, AND CONTAINING TAMOXIFEN
AGENT POUR LE TRAITEMENT DES TUMEURS A BASE DE LIPOSOMES ET CONTENANT DU TAMOXIFENE

(30) Priorität: 04.12.1998 DE 19855953
(43) Veröffentlichungstag der Anmeldung: 26.09.2001
(73) Patentinhaber: MAX-DELBRÜCK-CENTRUM FÜR MOLEKULARE MEDIZIN, 13125 Berlin (DE)
(72) Erfinder: ZEISIG, Reinhard, D-10435 Berlin (DE); FICHTNER, Iduna, D-13125 Berlin (DE); ARNDT, Dietrich, D-12589 Berlin (DE)
(74) Vertreter: Baumbach, Friedrich, Dr.
(86) Internationale Anmeldenummer: DE9903952
(87) Internationale Veröffentlichungsnummer: WO00033917

(56) Entgegenhaltungen:
- EP-A- 0 152 379
- EP-A- 0 359 981
- WO-A-00/01392
- WO-A-99/59599
- DE-C- 4 408 011
- R. ZEISIG: "Increase of the antitumor effect of liposomal octadecyl-piperidinoyl-phosphate: influence of composition on the final effect" HTTP://WWW.MICROB.UNI.WROC.PL/BIOCHEM/ZEIS IG.HTM, [Online] XP002136791 Retrieved from the Internet: <URL:http://www.microb.uni.wroc.pl/biochem /zeisig.htm> [retrieved on 2000-04-27]
- MAYER, LAWRENCE D. ET AL: "The role of tumor-associated macrophages in the delivery of liposomal doxorubicin to solid murine fibrosarcoma tumors" J. PHARMACOL. EXP. THER. (1997), 280(3), 1406-1414 , XP000905641
- SPRUSS T. ET AL: "Antitumor activity of miltefosine alone and after combination with platinum complexes on MXT mouse mammary carcinoma models." JOURNAL OF CANCER RESEARCH AND CLINICAL ONCOLOGY, (1993) 119/3 (142-149). , XP000905599
- BERGER M. R. ET AL: "Structure activity relationships if iv injectable alkylphosphocholines" PROCEEDINGS OF THE ANNUAL MEETING OF THE AMERICAN ASSOCIATION FOR CANCER RESEARCH,US,PHILADELPHIA, AACR, Bd. MEETING 87, 1996, Seite 387 XP000853551 ISSN: 0197-016X

## Beschreibung

Die Erfindung betrifft ein pharmazeutisches Mittel auf der Basis einer Kombination von Antiöstrogen, Alkylphospholipid und Phospholipiden, seine Herstellung und Verwendung. Anwendungsgebiete der Erfindung sind die Medizin und die pharmazeutische Industrie.

In der medikamentösen Tumortherapie ist vielfach durch das Auftreten von Resistenz gegen das Pharmakon und durch toxische Nebenwirkungen eine optimale Behandlung behindert. Ein Teil dieser unerwünschten Effekte läßt sich durch Verkapseln der Medikamente in Liposomen aufheben, bzw. mindern (D. D. Lasic und D. Papahadjopoulos, Medical Applictions of Liposomes, Elsevier, 1998). Liposomale Anthrazykline haben das Stadium der erweiterten klinischen Anwendung erreicht. Besondere Vorteile ergeben sich, wenn zur Liposomenbildung Phospholipide mit inhärenter Antitumorwirkung benutzt werden, z.B. Alkylphospholipide (Arndt et al. Drugs of Today 1998, 34, 83-96).

Alkylphospholipide sind neuartige Verbindungen, deren Wirkung gegen das Tumorwachstum durch Effekte auf die Zellmembran zustande kommt (Alkylphosphocholines: An update, Drugs of Today, Vol. 34, Suppl. F, 1998). Unter bestimmten Bedingungen ergeben Alkylphospholipide supramolekulare Strukturen, u.a. Liposomen, mit günstigeren Eigenschaften im Vergleich zur monomeren, bzw. micellaren Verbindung (DE 41 32 345 A1, DE 44 08 011 C1). In diese Liposomen mit inhärenter Antitumorwirkung können weitere Substanzen mit antineoplastischer Aktivität eingeschlossen werden (Arndt et al., Breast Cancer Res. Treatm. 43 (1997) 237-246, DE 44 08 011 C1).

Mammakarzinome, der häufigste Tumor der Frau, lassen sich in ca. 75% der Fälle durch endokrine Maßnahmen beeinflussen. Dabei hat insbesondere die kompetitive Hormontherapie mittels Tamoxifen Bedeutung, bei der am Rezeptor die endogenen Hormone antagonisiert werden. Die nebenwirkungsarme Tamoxifen-Behandlung wird jedoch durch die Entwicklung von Resistenz gegen das Pharmakon eingeschränkt. Ursachen der Resistenz sind u.a. Veränderungen am Ligand und dessen Bindung an den Östrogenrezeptor (ER), Verlust oder Veränderung des ER, Veränderungen von Transkriptionsfaktoren oder des ERassoziierten Proteins oder die Blockierung durch Antiöstrogen-Bindungsproteine (Katzenellenbogen et al., Breast Cancer Res. Treat. 44 (1997) 23-38; Osborne, New Engl. J. Med. 339 (1998) 1609-18; US005904930A).

Ziel der Erfindung ist die Schaffung einer Arzneimittelzusammensetzung auf der Basis von Antiöstrogen, Alkylphospholipid und Phospholipiden, das in Antiöstrogen-resistenten Tumoren wirksam ist, bzw. die Resistenzentwicklung minimiert oder verhindert.

Die Erfindung wird durch den Hauptanspruch charakterisiert, die Unteransprüche sind Vorzugsvariaten.

Wesentliches Merkmal der Erfindung ist die Kombination von antineoplastisch wirksamen Alkylphospholipid und einem Antiöstrogen in einem Lipidvesikel. Ein bevorzugtes Beispiel ist Octadecyl-(N,N-dimethylpiperidin-4-yl)-phosphat (OPP), Tamoxifen (Tam) in Phosphocholin (PC)-Vesikeln.

Im einzelnen ist das erfindungsgemäße Mittel durch folgende Zusammensetzung charakterisiert:
- ein Alkylphospholipid (mit antineoplastischer Wirksamkeit)
- ein wasser- oder lipidlösliches Antiöstrogen mit antineoplastischer Wirksamkeit
- ein antineoplastisch inertes Phospholipid
- ggf.Cholesterol oder ein anderes geeignetes Sterol
- ggf. ein Lipid mit positiver oder negativer Oberflächenladung
- ggf. ein polyethylenglycolmodifiziertes Lipid (PEG-Lipid)
- ggf. weitere Wirkstoffe und pharmazeutisch übliche Träger- und Hilfsstoffe.

Als Phospholipid-Analoga werden Alkylphospholipide mit Antitumorwirkung der allgemeinen Struktur I eingesetzt.

Struktur I: **R-Y-P-X**

In dieser Formel bedeuten
R: einen Alkyl-, Alkenyl-, oder Alkinylrest mit 12 bis 22 C-Atomen
Y: Sauerstoff, Schwefel oder CH₂
P: Phosphatgruppe (PO₂)
X: einen Cholin- oder modifizierten Cholinrest, oder Serin-, Ethanolamin-, Glycerin-gruppen, oder synthetische Modifikationen dieser Gruppen wie die Piperidin-4-yl-Gruppe

Bevorzugte Verbindungen sind Hexadecylphosphocholin, Octadecylphosphocholin, Erucylphosphocholin, Octadecyl-[2-(N-methylpiperidinio)ethyl]-phosphat, Octadecylphosphoethanolamin und Hexadecylphosphoserin.
Das mit den Phospholipid-Analogen assoziierte wasser- oder lipidlösliche Antiöstrogen wird durch Tamoxifen, Droloxifene, Toremifene, Idoxifene, Raloxifene, Miproxifene-Phospat (TAT-59), ICI 1643,384, ICI 182,780 und die Hauptmetabolite des Tamoxifens, 4-Hydroxytamoxifen und N-Desmethyltamoxifen, repräsentiert.

Phospholipide ohne eigenen antineoplastischen Effekt sind Lipide aus natürlichen Quellen oder synthetischer Herkunft wie sie üblicherweise zur Liposomenherstellung verwendet werden, z.B. Phosphatidylcholin.
Als PEG-Lipid wird bevorzugt polyethylenglycolmodifiziertes Phosphatidylethanolamin im Molekulargewichtsbereich von 1000 - 6000 Dalton eingesetzt. Es eignen sich unter anderem 1,2-Distearoyl-s,n-glycero-3-phosphoethanolamin-N-polyethylenglycol, MG∼2700; (PEG₂₀₀₀DSPE) und 1,2-Dipalmitoyl-sn-glycero-3-phosphoethanolamin-N-polyethylenglycol, MG∼5750 (PEG₅₀₀₀DPPE). Vorteilhaft ist auch der Einsatz von Verbindungen, die gleichzeitig PEG-Lipid und antineoplastisch wirksames Phospholipid-Analoges sind, wie Hexadecylphosphoethanolamin-N-polyethylenglycol.
Vorzugsweise wird gemäß der Erfindung ein antineoplastisch inertes Lipid natürlichen oder synthetischen Ursprungs als Basislipid für die Membranbildung eingesetzt, wie Phosphocholin, -serin, -ethanolamin, -glycerol oder andere ähnliche Lipide, wobei das Verhältnis Lipid zum Antiöstrogen 0-10 : 1 (Massenverhältnis: m/m) beträgt. Bevorzugt ist Cholesterol oder ein anderes geeignetes Sterol wie Sitosterol enthalten, wobei das Sterol zum Alkylphospholipid im Molverhältnis 0-1 : 1 steht.
Die liposomale Form besteht bevorzugt aus einschichtigen oder mehrschichtigen Vesikeln oder die Liposomen liegen als "reverse evaporation vesicles" vor.
Die resistenzüberwindende Wirkung des erfindungsgemäßen Mittels läßt sich in vitro und in vivo belegen.
Das erfindungsgemäße Mittel zur Tumortherapie ist pharmazeutisch stabil, physiologisch hervorragend verträglich und insbesondere zur intravenösen Applikation geeignet. Unerwünschter Metabolismus der Antiöstrogene wird vermieden, bzw. vermindert, es wird eine verbesserte Resorption und Verteilung des Pharmakons erreicht. In Wasser schwerlösliche Antiöstrogene sind in liposomaler Form gut applizierbar.
Das Mittel ist deshalb für eine Anwendung in der Tumortherapie hervorragend geeignet.

Die Erfindung wird durch folgende Beispiele erläutert:

### Beispiel 1:

4,62 mg Octadecyl-(1,1-dimethyl-piperidino-4-yl)-phosphat (OPP; 10 µmol), 0,387 mg Z-4-Hydroxy-Tamoxifen (HO-Tam, 1 µmol), 1,55 mg Cholesterol (4 µmol), und 1,1 mg Dicetylphosphat (DCP; 2 µmol) werden in 25 ml Chloroform/Methanol (7/3; v/v) vollständig gelöst, und dann das Lösungsmittel am Rotationsverdampfer vollständig abgedampft. Der erhaltene, fein verteilte Lipidfilm wird mit 1 ml phosphatgepufferter Kochsalslösung (PBS, pH 7,4) resuspendiert, nach Zugabe von einigen Glasperlen mindestens 3 Stunden bei Raumtemperatur auf der Schüttelmaschine intensiv bewegt. Die erhaltene Suspension aus multischichtigen Vesikel (MLV) wird dann duch Polykarbonatfilter, Porendurchmesser 100 nm mit einem LiposoFast Basis-System (Avestin, Inc. Ottawa, Canada) mehrfach extrudiert, bis Vesikel mit einem durchschnittlichen Durchmesser um 100 nm mit einer unimodaler Größenverteilung und einem Polydispersitätsindex kleiner 0,2 (Dynamische Lichtstreuungsmessung, DLS) erhalten werden.
Der Gehalt an OPP, HO-Tam, CH und DCP wird mittels HPTLC kontrolliert. Es werden etwa 85 % der Ausgangsmenge erhalten. Die Liposomen sind in der Zusammensetzung gegenüber der Ausgangszusammensetzung (Abweichung < 5%) unverändert. Diese HO-Tam-Liposomen werden vorzugsugsweise für in vitro-Untersuchungen eingesetzt.

### Beispiel 2:

36 mg OPP, 72 mg Tamoxifen-Citrat (Tam), 144 mg Phosphatidylcholin (PC) und 8,5 mg DCP werden in 100 ml Chloroform/Methanol (7/3; v/v) vollständig gelöst, und dann das Lösungsmittel am Rotationsverdampfer vollständig abgedampft. Der erhaltene, fein verteilte Lipidfilm wird mit 12 ml Zitronensäure/Phosphat-Puffer (pH 6.08) resuspendiert, nach Zugabe von einigen Glasperlen mindestens 3 Stunden bei Raumtemperatur auf der Schüttelmaschine intensiv bewegt. Es wird eine MLV-Suspension erhalten; die heterogen in ihrer Größenzusammensetzung ist mit Vesikeldurchmesser zwischen 100 und 5000 nm.
Diese Tam-Liposomen werden vorzugsugsweise für in vitro-Untersuchungen und als Ausgangsliposomen für Vesikel definierter Größe eingesetzt.

### Beispiel 3

36 mg OPP, 72 mg Tamoxifen-Citrat (Tam), 144 mg Phosphatidylcholin (PC) und 8,5 mg DCP und zusätzlich 9,7 mg N-(O-methyl-polyethylenglycyl)-1,2-distearyl-s,n-glycero-3-phosphoethanolamin (PEG₂₀₀₀DSPE) werden in 100 ml Chloroform/Methanol (7/3; v/v) vollständig gelöst, und dann das Lösungsmittel am Rotationsverdampfer vollständig abgedampft. Der erhaltene, fein verteilte Lipidfilm wird mit 12 ml Zitronensäure/Phosphat-Puffer (pH 6.08) resuspendiert, nach Zugabe von einigen Glasperlen mindestens 3 Stunden bei Raumtemperatur auf der Schüttelmaschine intensiv bewegt. Es wird eine MLV-Suspension erhalten; die heterogen in ihrer Größenzusammensetzung ist mit Vesikeldurchmesser zwischen 100 und 5000 nm. Diese Tam-Liposomen werden vorzugsugsweise für in vitro-Untersuchungen und als Ausgangsliposomen für Vesikel definierter Zusammnesetzung eingesetzt.

### Beispiel 4:

Tam-MLV aus Beispiel 2 werden duch Polykarbonatfilter, Porendurchmesser 200 nm mit einem LiposoFast Basis-System (Avestin, Inc. Ottawa, Canada) mehrfach extrudiert, bis eine unimodale Größenverteilung um 180 nm mit einem Polydispersitätsindex kleiner 0,35 (Dynamische Lichtstreuungsmessung, DLS) erhalten wird.

Der Gehalt an OPP, Tam, CH und DCP wird mittels HPTLC kontrolliert Es wird eine Liposomensuspension erhalten, die etwa 75 % an eingesetztem Tam und 98 % an OPP enthält. Darüber hinaus sind die Liposomen in der Zusammensetzung gegenüber der Ausgangszusammensetzung (Abweichung < 5%) unverändert. Die Tam-Liposomen werden vorzugsugsweise für in vivo Untersuchungen eingesetzt.

### Beispiel 5:

Peg-Tam-MLV aus Beispiel 3 werden duch Polykarbonatfilter, Porendurchmesser 200 nm mit einem LiposoFast Basis-System (Avestin, Inc. Ottawa, Canada) mehrfach extrudiert, bis eine unimodale Größenverteilung um 185 nm mit einem Polydispersitätsindex kleiner 0,33 (Dynamische Lichtstreuungsmessung, DLS) erhalten wird.
Der Gehalt an OPP, Tam, DCP und Peg₂₀₀₀DSPEwird mittels HPTLC kontrolliert Es wird eine Liposomensuspension erhalten, die etwa 75 % an eingesetztem Tam und 98 % an OPP enthält. Darüber hinaus sind die Liposomen in der Zusammensetzung gegenüber der Ausgangszusammensetzung (Abweichung < 5%) unverändert. Die Peg-Tam-Liposomen werden vorzugsugsweise für in vivo Untersuchungen eingesetzt.

### Beispiel 6:

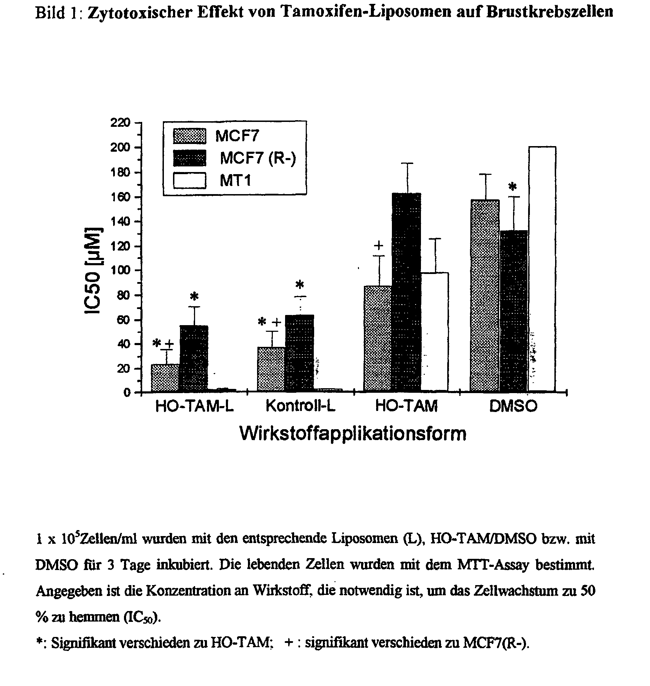

HO-Tam-Liposomen aus Beispiel 1 werden mit RPMI-Medium mit 10% fötales Kälberserum (ohne Indikatorzusatz, mit Adriamycin/Streptomycin), so verdünnt, daß eine Konzentration von 200 nmol/ml an OPP eingestellt ist, die dann weiter seriell verdünnt wird bis zu 0,78 nmol/ml. Die Konzentration an Wirkstoff HO-Tam beträgt dann entsprechend 20 nmol/ml bis 0,08 nmol/ml.
Die Brustkrebszellen MCF7, die sensitiv gegenüber Tamoxifen, sind und MCF7-R, die gegenüber dem Antiöstrogen resistent sind, werden in Mikrotiterplatten zu 2x10⁴ Zellen/well eingesät und am darauffolgenden Tag mit HO-Tam-Liposomen, Kontrollliposomen der Zusammensetzung wie die HO-Tam-Liposomen, jedoch ohne HO-Tam, HO-Tam, gelöst in DMSO und DMSO in gleicher Menge, wie zum Lösen des HO-Tam benötigt wird, für 3 Tage inkubiert. Danach werden die Überstände abgenommen, die Zellen mit PBS gewaschen, und dann die Zellwachstumshemmung mit dem MTT-Assay bestimmt. Dazu werden die Zellen mit 200 µl MTT-Lösung (4,6-Dimethylthiozol-2-yl-2,5-diphenyl-tetrazolium; 0,5 mg/ml) bei 37°C für 4 Stunden inkubiert, 170 µl des Überstandes vorsichtig abgehoben und die ausgefallenen Formasankristalle mit einer 70% Isopropanol-Lösung durch intensives Pipettieren und Schütteln vollständig in Lösung gebracht. Anschließend werden die Mikrotiterplatten photospektroskopisch bei 540 nm vermessen und die Wachstumshemmung im Vergleich zum Wachstum unbehandelter Zellen berechnet. Man erhält eine Wachstumshemmung, die im Bild 1 wiedergegeben ist.

### Beispiel 7:

Für die in vivo-Testung werden Tam-Liposomen nach Beispiel 4 eingesetzt. Als Tumormodell wird der Brustkrebs 3366/Tam auf weibliche NMRI-Nude-Mäusen transplantiert und die Behandlung begonnen, wenn der Tumor fühlbar ist. Die Tiere erhalten 2 x wöchentlich eine Dosis Liposomen mit 50 mg/kg Tam (und entsprechend 25 mg/kg OPP) über 4 Wochen. Als Kontrollen werden Liposomen verabreicht, die kein Tam enthalten, und außerdem noch eine Gruppe mit freiem Tam behandelt. Das Tumorwachstum im Verhältnis zur Kontrollgruppe (Physiologische Kochsalzlösung) wird bestimmt und als T/C Wert prozentual in Tabelle 1 dargestellt.

**Tabelle 1:**

| **Therapeutische Wirksamkeit von Tamoxifen-Liposomen gegenüber dem resistenten Brustkrebstumor 3366/Tam** | | | | |
|---|---|---|---|---|
| **Gruppe** | **Substanz** | **Dosis Tam/Lipid** | **Körpergewichtsveränderung** | **T/C** |
| | | mg/kg/Injektion | % (Tag 29/51) | % |
| A | Lösungsmittel | | 3 | |
| B | Tamoxifen | 50/0 | -5 | 91 |
| C | Tamoxifen-Liposomen | 50/25 | -5 | 63* |
| D | Kontroll-Liposomen | 0/25 | -4 | 88 |

| | | | | |
|---|---|---|---|---|
| * Signifikant verschieden zu Tamoxifen und zur Lösungsmittelkontrolle (p< 0,05) | | | | |

## Patentansprüche

1. Mittel zur Tumortherapie auf Liposomenbasis enthaltend:
- ein Alkylphospholipid mit antineoplastischer Wirksamkeit
- ein oder mehrere wasser- oder lipidlösliche Antiöstrogene mit antineoplastischem Effekt, das oder die mit dem Alkylphospholipid in liposomaler Form assoziiert ist (sind).
- ein Phospholipid ohne antineoplastische Wirksamkeit
- ggf. Cholesterol oder ein anderes geeignetes Sterol.
- ggf. ein Lipid mit positiver oder negativer Ladung
- ggf. ein polyethylenglycolmodifiziertes Lipid (PEG-Lipid)
- pharmazeutisch übliche Träger- und Zusatzstoffe.

2. Mittel nach Anspruch 1, **dadurch gekennzeichnet, daß** als Alkylphospholipid Verbindungen der allgemeinen Struktur I eingesetzt werden,
Struktur I: **R-Y-P-X**
wobei bedeuten:
R: einen Alkyl-, Alkenyl-, oder Alkinykest mit 12 bis 22 C-Atomen
Y: Sauerstoff, Schwefel oder CH₂
P: Phosphat (PO₂)
X: einen Cholin- oder modifizierten Cholinrest, oder Serin-, Ethanolamin-, Glycerin-gruppen, oder synthetische Modifikationen dieser Gruppen wie die Piperidin-4-yl-Gruppe.

3. Mittel nach Anspruch 1 und 2, **dadurch gekennzeichnet, daß** bevorzugt Hexadecylphosphocholin, Octadecylphosphocholin, Erucylphosphocholin, Octadecyl-[2-(N-methylpiperidino)ethyl]phosphat, Octadecylphosphoethanolamin und Hexadecylphosphoserin verwendet werden.

4. Mittel nach Anspruch 1, **dadurch gekennzeichnet, daß** als weitere antineoplastisch wirksame Substanzen, die mit dem Alkylphospholipid in liposomaler Form assoziiert sind, Antiöstrogene wie Tamoxifen, Droloxifen, Toremifene, Idoxifene, Raloxifene, Miproxifene-Phosphat (TAT-59), ICI 164,384 (N-n-Butyl-N-methyl-11-(3,17β-dihydroxyestra-1,3,5(10)-trien-7α-yl)undecamid) und ICI 182,780 (Faslodex®. 9-(4,4,5,5,5-Pentafluorpentylsulphinyl-3,17β-dihydroxyestra-1,3,5(10)-trien-7α-yl)-nonan), und die Hauptmetabolite des Tamoxifens, 4-Hydroxytamoxifen, N-Desmethyltamoxifen verwendet werden.

5. Mittel nach Anspruch 1, **dadurch gekennzeichnet, daß** ein antineoplastisch inertes Lipid natürlichen oder synthetischen Ursprungs als Basislipid für die Membranbildung eingesetzt wird wie Phosphocholin, -serin, -ethanolamin, -glycerol oder andere ähnliche Lipide, wobei das Verhältnis Lipid zum Antiöstrogen 0-10 : 1 (m/m) beträgt.

6. Mittel nach Anspruch 1, **dadurch gekennzeichnet, daß** Cholesterol oder ein anderes geeignetes Sterol wie Sitosterol enthalten ist und das Sterol zum Alkylphospholipid im Molverhältnis 0-1 : 1 steht.

7. Mittel nach Anspruch 1, **dadurch gekennzeichnet, daß** ein polyethylenglycolmodifiziertes Lipid (PEG-Lipid), vorzugsweise N-(O-methoxy-polyethylenglycyl)-1,2-distearyl-s,n-glycero-3-phosphoethanolamin (PEG₂₀₀₀-DSPE), zugesetzt wird.

8. Mittel nach Anspruch 1, **dadurch gekennzeichnet, daß** es als erste antineoplastisch wirksame Substanz Octadecyl-(N,N-dimethylpiperidin-4-yl)-phosphat (OPP) und als weitere antineoplastisch wirksame Substanz Tamoxifen enthält.

## Claims

1. Liposome-based composition for tumour therapy, comprising:
- an alkylphospholipid having anti-neoplastic activity
- one or more than one water- or lipid-soluble anti-oestrogen(s) having an anti-neoplastic effect, which is/are associated in liposomal form with the alkylphospholipid
- a phospholipid without anti-neoplastic activity
- optionally, cholesterol or any other suitable sterol
- optionally, a lipid having positive or negative charge
- optionally, a polyethylene-glycol-modified lipid (PEG lipid)
- pharmaceutically customary carriers and adjuvants.

2. Composition according to claim 1, **characterised in that** as alkylphospholipid there are used compounds of general structure I,
Structure I: **R-Y-P-X**
wherein:
R denotes: an alkyl, alkenyl or alkynyl radical having 12 to 22 C atoms
Y denotes: oxygen, sulphur or CH₂
P denotes: phosphate (PO₂)
X denotes: a choline or modified choline radical, or serine, ethanolamine or glycerol groups, or synthetic modifications of those groups such as the piperidin-4-yl group.

3. Composition according to claim 1 and 2, **characterised in that** preferably hexadecylphosphocholine, octadecylphosphocholine, erucylphosphocholine, octadecyl[2-(N-methylpiperidino)ethyl] phosphate, octadecylphosphoethanolamine and hexadecylphosphoserine are used.

4. Composition according to claim 1, **characterised in that**, as further anti-neoplastically active substances associated with the alkylphospholipid in liposomal form, there are used anti-oestrogens such as tamoxifen, droloxifene, toremifene, idoxifene, raloxifene, miproxifene phosphate (TAT-59), ICI 164,384 (N-n-butyl-N-methyl-11-(3,17β-dihydroxyestra-1,3,5(10)trien-7α-yl)undecamide) and ICI 182,780 (Faslodex®. 9-(4,4,5,5,5-pentafluoropentylsulphinyl-3,17β-dihydroxyestra-1,3,5(10)trien-7α-yl)nonane) and the principal metabolites of tamoxifen, 4-hydroxytamoxifen and N-desmethyltamoxifen.

5. Composition according to claim 1, **characterised in that** an anti-neoplastically inert lipid of natural or synthetic origin such as phospho-choline, -serine, -ethanolamine, -glycerol, or other similar lipids, is used as base lipid for membrane formation, the ratio of lipid to anti-oestrogen being 0-10 : 1 (m/m).

6. Composition according to claim 1, **characterised in that** cholesterol or another suitable sterol such as sitosterol is included and the sterol is in a molar ratio of 0-1 : 1 to the alkylphospholipid.

7. Composition according to claim 1, **characterised in that** a polyethylene-glycol-modified lipid (PEG lipid), preferably N-(O-methoxy-polyethylene glycyl)-1,2-distearyl-s,n-glycero-3-phosphoethanolamine (PEG₂₀₀₀-DSPE), is added.

8. Composition according to claim 1, **characterised in that** it comprises octadecyl-(N,N-dimethylpiperidin-4-yl) phosphate (OPP) as first anti-neoplastically active substance and tamoxifen as further anti-neoplastically active substance.

## Revendications

1. Agent pour le traitement des tumeurs à base de liposomes contenant :
- un alkylphospholipide ayant une efficacité antinéoplasique
- un, ou plusieurs anti-oestrogènes solubles dans l'eau ou les lipides avec un effet antinéoplasique, qui est (sont) associé(s) sous forme liposomiale avec l'alkylphospholipide,
- un phospholipide sans activité antinéoplasique,
- éventuellement du cholestérol ou un autre stérol approprié,
- éventuellement un lipide avec une charge positive ou négative,
- éventuellement un lipide modifié par un polyéthylèneglycol (PEG-lipide),
- des véhicules et additifs pharmaceutiquement acceptables.

2. Agent selon la revendication 1, **caractérisé en ce qu'**on utilise comme alkylphospholipide des composés de structure générale I,
Structure I ; R-Y-P-X
dans laquelle :
R représente un groupe alkyle, alcényle ou alcynyle ayant 12 à 22 atomes de carbone,
Y représente l'oxygène, le soufre ou CH₂
P représente le phosphate (PO₂)
X représente un groupe choline ou choline modifiée, ou des groupes sérine, éthanolamine, glycérine, ou des modifications synthétiques de ces groupes comme le groupe pypéridine-4-yle.

3. Agent selon la revendication 1 et 2, **caractérisé en ce qu'**on utilise de préférence l'hexadécylphosphocholine, l'octadécylphosphocholine, la érucylphosphocholine, l'octadécyl-[2-N-méthylpipéridino)éthyle]phosphate, l'octadécylphosphoéthanolamine et l'hexadécylphosphosérine.

4. Agent selon la revendication 1, **caractérisé en ce qu'**on utilise comme autres substances actives antinéoplasiques, qui sont associées avec l'alkylphospholipide sous forme liposomiale, des antioestrogènes comme le tamoxifène, le droloxifène, le torémifène, l'idoxifène, le raloxifène, le miproxifène-phosphate (TAT-59), ICI 164 384 (N-n-butyl-N-méthyl-11-(3,17β-dihydroxyestra-1,3,5-(10)-trièn-7α-yl)-undécamide) et ICI 182 780 (Faslotex® 9-(4,4,5,5,5-pentafluoropentylsulfinyl-3,17β-dihydroxyestra-1,3,5(10)-trièn-7-α-yl)-nonane), et les métabolites principaux du tamoxifène, 4-hydroxytamoxifène, N-desméthyltamoxifène.

5. Agent selon la revendication 1, **caractérisé en ce qu'**on utilise un lipide antinéoplasique inerte d'origine naturelle ou synthétique comme lipide de base pour la formation de membrane comme la phosphocholine, -sérine, -éthanolamine, -glycérol ou d'autres lipides analogues, dans lequel le rapport lipide sur antioestrogène s'élève à 0-10 : 1 (m/m).

6. Agent selon la revendication 1, **caractérisé en ce qu'**il contient du cholestérol ou un autre stérol approprié comme le sitostérol et que le styrol est dans le rapport molaire par rapport à l'alkylphospholipide de 0-1 : 1.

7. Agent selon la revendication 1, **caractérisé en ce qu'**on ajoute un lipide modifié par du polyéthylèneglycol (PEG-lipide), de préférence la N-(O-méthoxypolyéthylèneglycyl)-1,2-distéaryl-s,n-glycéro-3-phosphoéthanolamine (PEG₂₀₀₀-DSPE).

8. Agent selon la revendication 1, **caractérisé en ce qu'**il contient comme première substance antinéoplasique active l'octadécyl-(N,N-diméthylpipéridine-4-yle)-phosphate (OPP) et comme autre substance néoplasique active le tamoxifène.
